# EUROPEAN PATENT APPLICATION

(11) **EP 1 743 620 A1**
(43) Date of publication of application: **17.01.2007**
(21) Application number: 04729742.9
(22) Date of filing: 27.04.2004
(51) Int. Cl.: A61K 8/18

(54) **COSMETIC HAIR PREPARTION COMPOSITION**

(71) Applicant: Takasago International Corporation, Tokyo-to 144-8721 (JP)
(72) Inventor: SHIROYAMA, Kenichiro, Takasago International Corp., Hiratsuka-shi, Kanagawa 254-0073 (JP); KUMAMOTO, Hiroyasu, Takasago International Corp., H iratsuka-shi, Kanagawa 254-0073 (JP); HIRAMOTO, Tadahiro, Takasago International Corp., Hiratsuka-shi, Kanagawa 254-0073 (JP)
(74) Representative: Uchida, Kenji
(86) International application number: PCT/JP2004/006077
(87) International publication number: WO 2005/105021

(57) **Abstract**

The present invention relates to a hair cosmetic composition capable of treating hairs without causing damages to hairs and injuries of skins and having oxidation fixing effect of permanent wave and dyeing effect equal to or superior to those in a usual case. Specifically, it relates to a hair cosmetic composition comprising a polyphenol derivative obtainable by subjecting a polyphenol to a reaction using an alkaline solvent under the coexistence of oxygen molecules at a pH value during reaction of 6.5 or more.

## Description

### Technical Field

The present invention relates to a hair cosmetic composition. Specifically, it relates to a hair cosmetic composition comprising a polyphenol derivative obtainable by subjecting a polyphenol to a reaction using an alkaline solvent under the coexistence of oxygen molecules at a pH value during reaction of 6.5 or more. More specifically, it relates to a hair cosmetic composition comprising a polyphenol derivative obtainable by subjecting a specified polyphenol to a reaction using an alkaline solvent under the coexistence of oxygen molecules at a pH value during reaction of 6.5 or more, and a hair cosmetic composition comprising a polyphenol derivative obtainable by subjecting a specified polyphenol and amino acid to a reaction using an alkaline solvent under the coexistence of oxygen molecules at a pH value during reaction of 6.5 or more. Further, the present invention relates to a method of using a polyphenol derivative obtainable by subjecting a polyphenol to a reaction using an alkaline solvent under the coexistence of oxygen molecules at a pH value during reaction of 6.5 or more for the oxidation process required for the treatment of hairs.

### Background Art

In the permanent wave operation, a method of disconnecting S-S bond in hairs by reduction by a first agent comprising a reducing agent such as thioglycolic acid or cysteine as a main ingredient, followed by oxidatively fixing the hairs by a second agent comprising an oxidizing agent such as sodium borate, perborate, or hydrogen peroxide as a main ingredient has been generally adopted. Usually, hydrogen peroxide in the permanent second agent is at a concentration of from 1.0% by mass to 2.5% by mass in use.

Further, in the oxidizing hair dyeing agent, for oxidation polymerization of an oxidation dye contained in the hair dyeing agent and for discoloration of hairs, hydrogen peroxide, sodium perborate, sodium percarbonate, etc. have been used so far as an oxidizing agent. In this regard, the concentration of hydrogen peroxide in the hair dyeing agent is usually from 1.5% by mass to 4.0% by mass as the concentration in use.

However, use of such oxidizing agents involves a problem of causing damages to hairs, head hairs, skins and scalps or giving stimulation to skins or scalps.

In order to solve such problems, a hair dyeing agent containing an oxidase (JP-A-11-21215) and a permanent wave agent (Japan Cosmetic Technology Journal 24 (3), 220 (1991)) have been proposed. However, there has been a problem for the stability of the oxidase in the hair dyeing agent.

### Disclosure of the Invention

A subject of the present invention is to provide a hair cosmetic composition having a sufficient effect with a moderate oxidizing activity. Further, the present invention provides a hair cosmetic composition having an oxidative function such as an oxidizing hair dyeing agent and oxidatively fixing composition for permanent wave with less stimulations to skins and scalps, and less injuring head hairs, hairs, and skins or scalps.

The present inventors have made an earnest study for solving the foregoing subjects and, as a result, have found that a polyphenol derivative obtainable by subjecting a specified polyphenol compound to a reaction using an alkaline solvent under the coexistence of oxygen molecules at a pH during reaction of 6.5 or more has an oxidizing power. Further, they have found that a polyphenol derivative obtainable by subjecting a specified polyphenol compound and amino acid to a reaction using an alkaline solvent under the coexistence of oxygen molecules at a pH during reaction of 6.5 or more has an oxidizing power. Accordingly, they have found that hairs can be treated without causing damages to hairs and head hairs and injuries of skins and scalps by using the polyphenol derivative as a hair cosmetic composition, and then have completed the invention based on further various studies.

That is, the present invention relates to the followings.
(1) A hair cosmetic composition, which comprises a polyphenol derivative obtainable by subjecting a polyphenol to a reaction using an alkaline solvent under the coexistence of an oxygen molecule at a pH during reaction of 6.5 or more.
(2) The hair cosmetic composition according to (1), wherein the polyphenol is a polyphenol having an o-diphenol structure.
(3) The hair cosmetic composition according to (1), wherein the polyphenol is a polyphenol having a p-diphenol structure.
(4) The hair cosmetic composition according to any one of (1) to (3), wherein the polyphenol derivative is obtainable by further adding and reacting a divalent or multivalent metal ion.
(5) The hair cosmetic composition according to any one of (1) to (4), wherein the polyphenol derivative is a polyphenol derivative obtainable by subjecting a plant extract which contains a polyphenol and does not substantially contains an amino acid to a reaction using an alkaline solvent under the coexistent of an oxygen molecule at a pH value during reaction of 6.5 or more.
(6) The hair cosmetic composition according to any one of (1) to (5), wherein the polyphenol derivative is obtainable by further adding and reacting an amino acid.
(7) A hair cosmetic composition, which comprises a polyphenol derivative obtainable by subjecting a plant extract and/or plant body containing a polyphenol and an amino acid to a reaction using an alkaline solvent under the coexistent of an oxygen molecule at a pH value during reaction of 6.5 or more.
(8) The hair cosmetic composition according to any one of (1) to (7), wherein the hair cosmetic composition is an oxidative fixing composition for permanent wave.
(9) A method of treating hairs with a polyphenol derivative obtainable by subjecting a polyphenol to a reaction using an alkaline solvent under the coexistence of an oxygen molecule at a pH during reaction of 6.5 or more.
(10) A method of oxidatively fixing hairs during a permanent operation with a polyphenol derivative obtainable by subjecting a polyphenol to a reaction using an alkaline solvent under the coexistence of an oxygen molecule at a pH during reaction of 6.5 or more.

### Best Mode for Carrying Out of the Invention

The present invention is to be described in detail.

The invention relates to a hair cosmetic composition comprising a polyphenol derivative obtainable by subjecting a polyphenol to a reaction using an alkaline solvent under the coexistence of an oxygen molecule at a pH during reaction of 6.5 or more.

First, a polyphenol derivative obtainable by subjecting a polyphenol to a reaction using an alkaline solvent under the coexistence of an oxygen molecule at a pH during reaction of 6.5 or more is explained below.

Polyphenol used in the invention means a compound having two or more phenolic hydroxyl groups in one identical molecule, and the polyphenol also includes glycosides thereof. The polyphenol used in the invention is not particularly limited so long as it is a polyphenol capable of attaining the aimed object.

Specific examples of such polyphenol include apigenin, apigenin glycoside, acacetin, alkanin, isorhamnetin, isorhamnetin glycoside, isoquercitrin, epicatechin, epicatechin gallate, epigallocatechin, epigallocatechin gallate, esculetin, ethylprotocatechuate salt, ellagic acid, catechol, gamma acid, catechin, gardenin, gallocatechin, caffeic acid, caffeic acid ester, chlorogenic acid, kaempferol, kaempferol glycoside, quercetin, quercetin glycoside, quercetagenin, genistin, genistin glycoside, gossypetin, gossypetin glycoside, gossypol, shikonin, 4-dihydroxyanthraquinone, 1,4-dihydroxynaphthalene, cyanidin, cyanidin glycoside, sinensetin, diosmetin, diosmetin glycoside, 3,4'-diphenyldiol, sinapic acid, stearyl-β-(3,5-di-tert-butyl-4-hydroxyphenyl) propionate, spinacetin, tangeritin, taxifolin, tannic acid, daphnetin, tyrosine, delphinidin, delphinidin glycoside, teaflavin, teaflavin monogallate, teaflavin bisgallate, tricetinidin, dopa, dopamine, naringenin, naringin, nordihydro guaiaretic acid, noradrenalin, hydroquinone, vanillin, patchouletin, herbacetin, vanillyl alcohol, vanitrope, vanillin propylene glycol acetal, vanillic acid, bis(4-hydroxyphenyl) sulfonic acid, bisphenol A, pyrocatechol, vitexin, 4,4'-biphenyldiol, 4-tert-butyl catechol, 2-tert-butylhydroquinone, protocatechuic acid, phloroglucinol, phenolic resin, procyanidin, prodelphinidin, phloretin, phloretin glycoside, fisetin, folin, fervasetin, fraxetin, purpurin, phloridin, peonidin, peonidin glycoside, pelargonidin, pelargonidin glycoside, petunidin, petunidin glycoside, hesperetin, hesperidin, gallic acid, gallic acid ester (such as lauryl gallate, propyl gallate and butyl gallate), mangeferin, malvidin, malvidin glycoside, myricetin, myricetin glycoside, 2,2'-methylenebis(4-methyl-6-tert-butylphenol), 2,2'-methylenebis(4-ethyl-6-tertbutylphenol), methyl atrarate, 4-methylcatechol, 5-methylcatechol, 4-methoxycatechol, 5-methoxycatechol, methylcatechol-4-carboxylic acid, 2-methylresorcinol, 5-methylresorcinol, morin, limocitrin, limocitrin glycoside, limocitrol, luteolin, luteolin glycoside, luteolinidin, luteolinidin glycoside, rutin, resorcin, resveratrol, resorcinol, leucocyanidin, and leucodelphinidin.

Among these polyphenols, polyphenols having an o-diphenol structure, for example, flavonoids such as quercetin, epicatechin, and epigallocatechin and glycosides thereof, gallic acid, gallic acid ester, chlorogenic acid, caffeic acid, caffeic acid ester, tannic acid, pyrocatechol, nordihydro guaiaretic acid, L-dopa, 4-methylcatechol, 5-methylcatechol, 4-methoxycatechol, and 5-methoxycatechol; and polyphenols having a p-diphenol structure such as hydroquinone, shikonin and homologues thereof, alkanin and homologues thereof, and purpurin.

The o-diphenol structure means a structure in which two hydroxyl groups are substituted directly on the benzene ring and the hydroxyl groups are in adjacent with each other. Further, the p-diphenol structure means a structure in which two hydroxyl groups are directly substituted on the benzene ring and the hydroxyl groups are present at para-positions.

The polyphenols may be used each alone or two or more of them may be used in combination.

The polyphenol described above can be prepared by conventional methods, or commercial products may also be purchased. Further, they may be prepared by synthesis. Further, polyphenol fractions at high concentration prepared from plants may also be used.

According to the invention, plant extracts containing polyphenols may also be used instead of the polyphenols described above. As the plant extracts in this case, plant extracts which contain polyphenol and do not substantially contain amino acid are preferred. As the plant extracts, those prepared by conventional methods may be used, or commercial products may be used. Examples of the plant extracts are shown below.

A polyphenol compound and a polyphenol-containing plant extract which does not substantially contains an amino acid may be used together.

The polyhenol derivative in the invention can be obtained also by subjecting a polyphenol and an amino acid to a reaction using an alkaline solvent under the coexistent of an oxygen molecule at a pH value during reaction of 6.5 or more.

While the amino acid used in the method described above is not particularly limited so long as the amino acid provides an aimed effect of the invention, an α-amino acid is particularly preferred among the amino acids. The α-amino acid means herein an amino acid in which one amino group and one carboxyl group are bonded to one identical carbon atom. Examples of the α-amino acid include glycine, alanine, valine, leucine, isoleucine, glutamic acid, aspartic acid, glutamine, asparagine, serine, threonine, lysine, hydroxylysine, alginine, histidine, cystine, methionine, phenylalanine, tyrosine, tryptophan, proline, 4-hydroxyproline, cysteine, theanine, amino acid salts (such as sodium glutamate and sodium aspartate).

Among them, glycine, alanine, glutamic acid, aspartic acid, lysine, alginine, histidine, serine, cystine, methionine, cystein, sodium glutamate, sodium aspartate, and tyrosine are preferred.

The amino acids can be available easily by purchasing commercial products. Further, the amino acids may be used each alone, or two or more of them may be used in combination. Further, a plant-extract containing an amino acid can also be used.

Further, according to the method described above, a plant extract which does not substantially contain a polyphenol and contains an amino acid can also be used instead of the amino acid. While the plant extract which does not substantially contain a polyphenol and contains an amino acid can be prepared by using conventional methods, commercial products may also be purchased. An amino acid and an amino acid-containing plant extract which does not substantially contain a polyphenol may also be used in combination.

In a case of obtaining the polyphenol derivative in the invention, examples using the polyphenol and the amino acid in combination include an example using an amino acid-containing plant extract which does not substantially contain a polyphenol and a polyphenol together, an example using a polyphenol-containing plant extract which does not substantially contain an amino acid and an amino acid together, and an example using a polyphenol-containing plant extract which does not substantially contain an amino acid and an amino acid-containing plant extract which does not substantially contain a polyphenol. Examples of plant extracts are shown below.

The amount of "not substantially containing" the amino acid or the polyphenol is such an amount as giving no effects on the reaction and this is an amount out of the detection limit when measured in accordance with a generally known method.

The ratio for the mixing amount of a polyphenol and an amino acid to be reacted upon obtaining a polyphenol derivative according to the invention is appropriately controlled depending on the polyphenol and the amino acid to be adopted. Generally, it is preferred that the polyphenol and the amino acid are mixed at a molar ratio of from 9:1 to 1:9 and it is more preferred that they are mixed at a ratio of from 3:1 to 1:3. The definition is specified for effectively utilizing the polyphenol and the amino acid when they are used as the starting substances and it does not exclude a case that either one of the both substances is present in a great amount.

Herein, the alkaline solvent is conventionally known and it is typically an alkaline substance-containing solvent formed by dissolving an alkaline substance to a solvent such as water.

The alkaline substances are not particularly limited and examples thereof include carbonates such as sodium carbonate, potassium carbonate, sodium bicarbonate, ammonium carbonate, and guanidine carbonate; hydrogen carbonates; borates such as potassium borate and sodium borate; silicates such as potassium silicate, sodium silicate No. 1, sodium silicate No. 2, sodium silicate No. 3, sodium orthosilicate, and sodium metasilicate; sodium monohydrogen phosphate; sodium sulfite; sodium hydroxide; calcium hydroxide; potassium hydroxide; magnesium hydroxide; ammonium hydroxide; sodium pyrophosphate; and potassium pyrophosphate.

As the solvent dissolving one or plurality of such alkaline substances, water and various water containing solvents can be mentioned as preferred solvents. Further, a so-called alkaline buffer using the alkaline substance described above and an acid may also be used as the solvent.

Although the solvents described above usually exhibit alkalinity with a pH value of 7.0 or more and they are alkaline before reaction, they sometime exhibit weak acidity depending on the substance coexistent in the solvent, such as starting material including polyphenol and amino acid, and the addition amount thereof. That is, the solvent before reaction is always alkaline and a preferred effect is obtained when the pH of the solvent in the reaction system after starting the reaction is 6.5 or more upon obtaining the polyphenol derivative described above. Particularly, it is preferred that the pH during reaction is within a range from 7 to 13 and, further, it is more preferred that the pH is within a range from 8 to 13. A polyphenol derivative having a preferred oxidizing activity, oxidatively fixing effect for permanent wave and dyeing effect can be obtained by defining the pH in the reaction system within the range described above during reaction.

According to the invention, it is necessary that the polyphenol is subjected to a reaction under the coexistence of an oxygen molecule. Convenient means for supplying the oxygen molecule into the reaction system include delivery of oxygen or air into the system by utilizing an air pump, etc. (i.e., bubbling), or positive stirring of the system. Reaction under the coexistence of the oxygen molecule means a reaction with an aim of enabling to positively introduce oxygen molecules into a reaction solution thereby proceeding the reaction of the polyphenol present in the reaction system. In this case, a polyphenol derivative can be obtained more efficiently by controlling the amount of oxygen to be supplied into the reaction solution, preferably, to 1 mg/L or more and, more preferably, to 2 mg/L or more. While the oxygen supply amount can be attained, for example, by positively blowing an oxygen gas, air or a mixture thereof into the reaction system (i.e., bubbling), it can also be attained by stirring the reaction solution under the reaction condition in which the oxygen gas or air can always be contacted.

The temperature during reaction is not particularly limited and the polyphenol derivative of the invention can be obtained so long as it is from 0°C to a solvent reflux temperature. It is preferred to conduct reaction at 0°C to 60°C, more preferably, 0°C to 40°C and, further preferably, 0°C to 25°C in view of the efficiency of forming the polyphenol derivative and for avoiding thermal decomposition of the resultant polyphenol derivative.

According to the invention, while the polyphenol takes place reaction even in a short time, it is preferably reacted for about several minutes (2 min) to 24 hours, more preferably, about 10 minutes to 9 hours and, further preferably, 10 minutes to 7 hours in view of practical use. During the reaction for preparing the polyphenol derivative, pressurization is not particularly necessary but pressure may be applied.

Further, when the reaction is conducted under the coexistence of divalent or multivalent metal ions or a metal salt releasing divalent or multivalent metal ions in a reaction system, a polyphenol derivative which is further excellent in oxidizing activity, oxidatively fixing effect for permanent wave, dyeing effect and stability can be obtained.

Examples of the divalent or multivalent metal ions include copper ion, zinc ion, calcium ion, magnesium ion, silver ion, aluminum ion, and manganese ion.

Further, examples of the compounds releasing the divalent or multivalent metal ions include the followings. For example, they include, copper compounds such as copper chloride, copper fluoride, copper sulfate, copper nitrate, copper hydroxide, copper citrate, copper gluconate, copper aspartate, copper glutamate, sodium copper chlorophyllin, and copper chlorophyll; zinc compounds such as zinc chloride, zinc fluoride, zinc sulfate, zinc nitrate, zinc hydroxide, zinc citrate, zinc gluconate, zinc aspartate, zinc glutamate, zinc phosphate, and zinc lactate; calcium compounds such as calcium chloride, calcium hydroxide, calcium citrate, calcium gluconate, calcium L-glutamate, calcium carbonate, calcium lactate, calcium pantothenate, calcium dihydrogen pyrophosphate, calcium propionate, calcium sulfate, tricalcium phosphate, calcium monohydrogen phosphate, calcium dihydrogen phosphate, calcium disodium ethylenediaminetetraacetate; magnesium compounds such as magnesium chloride, magnesium sulfate, magnesium hydroxide, magnesium L-glutamate, magnesium oxide, and magnesium carbonate; silver compounds such as silver oxide; aluminum compounds such as aluminum chloride, aluminum hydroxide, aluminum acetate, aluminum borate, aluminum phosphate, and aluminum sulfate; and manganese compounds such as permanganate, for example, potassium permanganate and manganese sulfate. Further, titanium compounds such as titanium dioxide may also be used.

The addition amount of the metal ions is properly controlled depending on the situation of the reaction and it is generally preferred to add them such that the concentration of the metal ions in the reaction solution is from 0.00001 mM to 100 mM and, more preferably, from 0.00005 mM to 10 mM and, further preferably, from 0.1 mM to 5 mM.

According to the invention, when a polyphenol and a amino acid are reacted under the coexistence of the oxygen molecule in an alkaline solvent to obtain a polyphenol derivative, a plant extract containing a polyphenol and an amino acid can be used instead of the polyphenol and the amino acid. The plant extract in this case is preferably a plant extract containing a polyphenol and an amino acid each at high concentrations. As the plant extracts, those prepared by conventional methods may be used or commercial products may also be used.

For example, the polyphenol derivative according to the invention can be obtained by adding a plant extract containing a polyphenol and an amino acid, that is, an extract from at least one part selected from leaves, stalks, roots and seeds (fruits) of plants to an alkaline solvent, controlling the pH of the reaction solution during reaction to 6.5 or more, and treating them at an oxygen supply amount of 1 mg/L or more, at a reaction temperature of from 0°C to a solvent reflux temperature for a reaction time of from several minutes to 24 hours. Examples of the alkaline substance and the solvents in this case include those described above and they are obtainable by controlling the reaction conditions, etc. within the range described above. In a case of using the plant extract containing the polyphenol and the amino acid instead of the polyphenol and the amino acid, at least one member selected from a polyphenol-containing plant extract which does not substantially contain an amino acid, an amino acid-containing plant extract which dose not substantially contain a polyphenol, a polyphenol, and an amino acid may also be used together. Further, examples of the plant extracts are shown below.

According to the invention, when a polyphenol derivative is obtained by subjecting the polyphenol and the amino acid to a reaction using an alkaline solvent under the coexistence of oxygen molecule at a pH value during reaction of 6.5 or more, a plant body containing a polyphenol and an amino acid may also be used instead of the polyphenol and the amino acid. The plant body in this case preferably contains the polyphenol and the amino acid at high concentrations.

For example, the polyphenol derivative according to the invention can be obtained also by adding a plant body containing a polyphenol and an amino acid, that is, at least one part selected from leaves, stalks, roots, and seeds (fruits)of plants to an alkaline solvent, controlling the pH of the reaction solution during reaction to 6.5 or more, and treating them at an oxygen supply amount of 1 mg/L or more, at a reaction temperature of from 0°C to a solvent reflux temperature for a reaction time of from several minutes to 24 hours. Examples of the alkaline substance and the solvents in this case include those described above and they are obtained by controlling the reaction conditions, etc. in the same manner as described above. As the plant body, plants exemplified for the plant extracts described below can be used. In a case of using the plant body containing the polyphenol and the amino acid instead of the polyphenol and the amino acid, at least one member selected from a polyphenol containing plant extract which does not substantially contain an amino acid, an amino acid-containing plant extract which does not substantially contain a polyphenol, a plant extract containing a polyphenol and an amino acid, a polyphenol and an amino acid may also be used together.

Examples of plant extracts include those extracted from aloe, anise seed, elderberry, eleutherococcus, plantago, olive, orange flower, all spice, oregano, valeriana fauriei, chamomile, capsicum pepper, cardamom, cassia, garlic, caraway seed, clove, cumin seed, burdock, cola nitida, coriander seed, Chinese gall, sweet potato, saffron, japanese pepper, juniper berry, cinnamon, potato, ginger, star-anise, St. John's wort, celery seed, savory, sesame, pie plant, tarragon, turmeric, thistle, anethum graveolens, nutmeg, nettle, hibiscus, hamamelis, birch, basil, bitter orange, fennel, primrose, fenugreek, verbena, bay laurel, hop, boldo, horseradish, poppy seed, gallnut, marigold, marrow, marjoram, mustard, milfoil, mint leaves, melissa, mace, linden, Gentiana scabra var. buergeri, rosehip, rosemary, Rosmarinus officinalis, sunflower seed, grape pericarp, apple, carrot leaf, banana, strawberry, apricot, peach, plum, pineapple, pear, persimmon, cherry, papaya, mango, avocado, melon, loquat, fig, kiwifruit, prune, blue berry, black berry, raspberry, cranberry, coffee beans, cacao beans, grape seed, grape fruits seed, pecan nuts, cashew nuts, chestnuts, coconut, peanuts, walnut, green tea leaf, black tea leaf, oolong tea leaf, tobacco, perilla leaf, thyme, sage, lavender, spearmint, peppermint, blessed thistle, hyssop, sweet basil, marigold, dandelion, artichoke, matricaria chamomilla, Agrimonia pilosa var. japonica, licorice, anise, yarrow, eucalyptus, wormwood, balm, Angelica pubescens, fenugreek, Anaheim pepper, fennel, chili pepper, coriander seed, caraway seed, fennel seed, ginger, horseradish, marjoram, common marjoram, mustard, parsley, pepper, tarragon,turmeric, wasabi, dill seed, and citrus fruit. The plant extracts may be used by combining two or more of them.

According to the invention, in a case of using the plant extract upon obtaining the polyphenol derivative, a polyphenol derivative at a high concentration can be obtained also by adsorbing a polyphenol or a polyphenol and an amino acid in the plant extract to a resin or the like in accordance with a customary method, followed by bringing the same into contact with an alkaline solvent and air or oxygen.

The reaction solution containing the polyphenol derivative obtained as described above may also be condensed further by using means such as column, filtration or solvent extraction.

According to the invention, for the polyphenol derivative as an effective deoxidation ingredient, a liquid ingredient is usually removed from the reaction solution by a conventional method such as concentration, filtration, vacuum drying or freeze-drying to obtain a solid polyphenol derivative.

Thus, a polyphenol derivative as an effective ingredient of the deoxidant composition of the invention is obtained.

The polyphenol derivative as the effective ingredient of the invention is often colored and the color thereof changes depending on the kind of the polyphenol as the starting material, absence or presence of the amino acid, the kind and the quantity ratio of the amino acid, etc. Further, since the density of color also changes depending on the reaction time and the pH, it cannot be generally defined.

For example, referring to chlorogenic acid, a reaction solution of a pale yellow color upon start of the reaction turns brown with lapse of time and then dark brown. In a case of quercetin, a reaction solution of a pale pink color upon start of the reaction increases redness with lapse of time and gradually turns deep wine red. In a case of gallic acid, a reaction solution of a pale yellow color upon start of the reaction is tinted with green with lapse of time and then turns dark green. In a case of pyrocatechol, a reaction solution of a pale pink color upon start of the reaction turns brown with lapse of time and then turns dark brown.

Further, in a case of selecting and reacting glycin as the amino acid, the reaction solution with chlorogenic acid is green, the reaction solution with (+)-catechin is red, the reaction solution with protocatechuic acid is red, the reaction solution with pyrocatechol is pale pink, the reaction solution with esculetin is brown, the reaction solution with hydroquinone is brown, the reaction solution with quercetin is red, and the reaction solution with gallic acid is dark green.

For the reaction of most of polyphenols or between polyphenol and amino acid, while the reaction solution has a pale color upon start of the reaction, the color of the reaction solution tends to be thick gradually with lapse of the reaction time and finally grown rich in dense color. The time in which the color of the reaction solution is thickened changes depending on the kind of the polyphenol, combination of the polyphenol and the amino acid, and the reaction condition. While it is about several minutes after starting the reaction, it may sometimes be about 20 min or about 30 min after starting.

The polyphenol derivative prepared in the invention plays a role as an oxidizing ingredient in the hair cosmetic composition. The polyphenol derivative is a mixture having various chemical structures and, for example, reaction products of a polyphenol as the starting material, polymerizates prepared from polyphenol, reaction products from polyphenol and amino acid and polymerization products prepared from polyphenol and amino acid also belong to the category of the polyphenol derivatives of the invention so long as they provides an intended effect of the invention.

The molecular weight of the obtained polyphenol derivative, that is, the molecular weight of the active ingredient in the hair cosmetic composition is preferably more than the molecular weight of the polyphenol or the sum of the molecular weight for the polyphenol and the amino acid as the starting material before reaction and is also 10,000 or less. More preferably, it ranges from the molecular weight of the polyphenol or the sum of the molecular weight for the polyphenol and the amino acids to 5,000.

The molecular weight of the polyphenol derivative can be measured by the following method. That is, a reaction solution containing the polyphenol derivative prepared by various methods described above is concentrated by centrifugal separation, and it is determined whether the concentrates pass through a filtration membrane having predetermined pores or remains on the filtration membrane, and the molecular weight can be determined corresponding to the pores of the filtration film where the concentrates remain on the filtration membrane. Commercial products may be used as the filtration membrane used herein.

For the hair cosmetic composition according to the invention, polyphenol derivatives obtained from different polyphenols, plant extracts, or plant bodies can be used in combination.

The content ratio of the polyphenol derivative in the hair cosmetic composition of the invention is preferably from 0.001 to 20.0% by mass based on the total mass of the composition. It is within a range, more preferably, of from 0.005 to 15.0% by mass and, further preferably, of from 0.01 to 10.0% by mass.

In a case of incorporating the polyphenol derivative of the invention into the hair cosmetic composition, it can be incorporated in a state of a reaction solution prepared with a polyphenol derivative.

The hair cosmetic composition of the invention means a composition to be used in the process of applying an oxidizing treatment to hairs. Specifically, it can be used, for example, as an oxidation fixing composition for use in permanent wave or a hair dyeing agent (also referred to as a hair dye).

Application of the hair cosmetic composition of the invention is to be described specifically below.

The oxidation fixing composition for use in permanent wave according to the invention means a composition having an effect of oxidatively fixing a disconnected S-S bond (disulfide bond) in hairs during a permanent wave operation. For example, in a usual case of a two-agent-type permanent agent, S-S bond in the hair is disconnected with a first agent and the disconnected S-S bond is then oxidatively fixed with a second agent. Accordingly, examples of the oxidation fixing composition for use in permanent wave include a permanent wave agent, particularly, a two-agent-type permanent wave second agent. Further, the oxidation fixing composition for use in permanent wave of the invention can be used solely or can be used in combination with a conventional permanent wave second agent.

In the case of the two-agent-type permanent agent, the permanent wave agent having an effect of disconnecting the S-S bond in the hair is usually contained in the first agent and contains a reducing agent, for example, at least one thio-compound and/or inorganic sulfite salt.

Examples of the thio-compound include thioglycolic acid and salts thereof, thio lactic acid and salts thereof, cysteine and hydrochloride salt thereof, homocysteine, cysteamine, N-acetyl cysteine, thioglycerine; ethanediol monothioglycolate, 1,2-propylene glycol monothioglycolate, 1,3-propnanediol monothioglycolate, and isomer mixtures thereof; 1,3-butanediol monothioglycolate, 1,4-butanediol monothioglycolate, and isomer mixtures thereof; ethanediol monothiolactate, 1,2-propanediol monothiolactate, 1,3-propanediol monothiolactate, 1,4-butanediol monothiolactate, and isomer mixtures thereof; monothioglycolate and monothiolactate of polyethylene glycol such as di-, tri-, and tetra-ethyleneglycol; monothiolactate and monothioglycolate of polypropylene glycol such as di-, tri-, and tetrapropylene glycol; glycerol monothiolactate; and thio acids and esters thereof. Further, these compounds may be used solely or a plurality of them can be used in combination.

Among the thio-compounds described above, thioglycolic acid, thiolactic acid, and salts thereof, particularly, ammonium salts and ethanol amine salts; cysteine and hydrochlorides thereof are preferred.

The inorganic sulfite salts include, for example, alkali sulfite salts and alkali hydrogen sulfite salts.

For the permanent wave agent containing the reducing agent of the invention, a dithioglycolic acid and salts thereof can further be incorporated as a reaction controller. The dithioglycolic acid and the salts thereof have an effect of controlling the reducing power of the reducing agent and, particularly, in a case of using thioglycolic acid or thioglycolic acid salt such as monoethanolamine thioglycolate as the reducing agent, it is excellent in the effect of controlling the reducing power of thioglycolic acid or thiglycolic acid salt due to the equilibrium reaction thereof. Examples of the dithioglycolic acid and salts thereof include diammonium dithioglycolate which is an ammonium salt.

Into the permanent wave agent of the invention, an alkalizing agent can be further incorporated. The alkalizing agent includes, for example, ammonia, monoethanolamine, ammonium carbonate, and ammonium hydrogen carbonate.

In the permanent wave agent for oxidatively fixing the disconnected S-S bond in the hairs according to the invention, for example, the second permanent wave agent in the case of the two-agent-type, the polyphenol derivative of the invention is preferably incorporated by from 0.001 to 20.0% by mass based on the total mass of the second permanent wave agent. It is within a range, more preferably, of from 0.005 to 15.0% by mass and, further preferably, of from 0.01 to 10.0% by mass.

In addition, purified water or the like is incorporated in the second permanent wave agent.

In the permanent wave agent of the invention, optional ingredient usually contained in the permanent wave agent may be incorporated in addition to those described above.

Examples of the optional ingredient include various kinds of surfactants such as anionic surfactants, cationic surfactant, amphoteric surfactants, and nonionic surfactants; viscosity improvers such as carboxymethyl cellulose, hydroxyethyl cellulose, hydroxypropyl cellulose, or salts thereof, pullulan or derivatives thereof, carrageenane, xanthane gum, carboxyvinyl polymer, and various kinds of alkylolamides; oil and fats such as paraffin, fatty acid ester, animal and plant oils, lanoline, cetyl alcohol, choresterol, olive oil, squalane and Vaseline; higher alcohols such as cetyl alcohol, stearyl alcohol, and oleyl alcohol; protein hydrolysis products or derivatives thereof derived from animals and plants such as collagen, keratin, soybean protein, and wheat protein; solvents such as isopropanol, benzyl alcohol, ethanol and water; pH controllers such as sodium hydroxide, sodium hydrogen carbonate, ammonium hydrogen carbonate, ammonium monohydrogen phosphate, phosphoric acid, hydrochloric acid, succinic acid, citric acid, sodium citrate, and sodium pyrrolinate; antiseptic agents such as paraoxybenzoate esters and sodium dehydro acetate; polyhydric alcohols (humectants) such as propylene glycol and glycerine; chelating agents such as ethylenediamine tetraacetic acid; hair protecting ingredients, stabilizers, anti-inflammatory agents, coloring agents, antioxidants, emlulsifiers, perfumes, etc.

The permanent wave agent of the invention may be in a formulation such as liquid, emulsion, cream, gel, paste, mousse, solid, powder or the like and can be formed also as an aerosol or spray.

The permanent wave treatment of the invention can be used for wave perming treatment, straight perming treatment or the like.

Then, description is to be made to a case of using the hair cosmetic composition of the invention as a hair dye.

The hair dyeing agent (hair dye) of the invention includes, for example, oxidizing hair dyeing agent. The form includes, for example, one-agent-type hair dyeing agent, and two-agent-type hair dyeing agent.

As the dye used for the hair dyeing agent of the invention, all of usual oxidation dyes can be used.

Examples of the oxidizing dye include resorcine, 5-aminoorthocresol, 3,3'-iminodiphenol, 2,4-diaminophenol hydrochloride, toluene-2,5-diamine hydrochloride, paraphenylenediamine hydrochloride, N-phenylparaphenylenediamine hydrochloride, methaphenylenediamine hydrochloride, orthoaminophenol, catechol, N-phenylparaphenylenediamine acetate, 2,6-diaminopyridine, 1,5-dihydroxynaphthalene, diphenylamine, toluene-2,5-diamine, toluene-3,4-diamine, α-naphthol, paraaminophenyl sulfamic acid, paraaminophenol, hydroquinone, pyrogallol, N-phenylparaphenylene diamine, phloroglucin, metha-aminophenol, metha-phenylene diamine, 5-aminoorthocresol sulfate, orthoaminophenol sulfate, orthochloroparaphenylene diamine sulfate, 4,4'-diaminodiphenylamine sulfate, toluene-2,5-diamine sulfate, paraaminophenol sulfate, paraphenylene diamine sulfate, paramethylaminophenol sulfate, methaaminophenol sulfate, methaphenylenediamine sulfate, 2,4-diaminophenoxyethanol hydrochloride, and 5-(2-hydroxyethylamino)-2-methyl phenol.

Further, 2-amino-4-nitrophenol, 2-amino-5-nitrophenol, 1-amino-4-methylamino-anthraquinone, nitroparaphenyl diamine hydrochloride, 1,4-diaminoanthraquinone, nitroparaphenylene diamine, paranitroorthophenylene diamine, picramic acid, 2-amino-5-nitrophenol sulfate, nitroparaphenylene diamine sulfate, paranitroorthophenylene diamine sulfate, paranitromethaphenylene diamine sulfate, etc. are usually used often in combination with the oxidizing dye.

The alkali agent incorporated into the hair dyeing agent of the invention includes alkanolamines such as monoethanol amine and isopropanol amine, guanidium salts such as guanidine carbonate, and ammonia.

For the hair dyeing agent of the invention, the polyphenol derivative of the invention is preferably contained by from 0.001 to 20.0% by mass based on the total mass of the hair dyeing agent. It is within a range, more preferably, of from 0.005 to 15.0% by mass and, further preferably, of from 0.01 to 10.0% by mass.

Into the hair dyeing agent of the invention, optional ingredients usually used in the field of cosmetics can be incorporated in addition to the ingredients described above.

Examples of the optional ingredient include various kinds of surfactants such as anionic surfactants, cationic surfactant, amphoteric surfactants, and nonionic surfactants; viscosity improvers such as carboxymethyl cellulose, hydroxyethyl cellulose, hydroxypropyl cellulose, or salts thereof, pullulan or derivatives thereof, carrageenane, xanthane gum, carboxyvinyl polymer, and various kinds of alkylolamides; oil and fats such as paraffin, fatty acid ester, animal and plant oils, lanoline, cetyl alcohol, choresterol, olive oil, squalane and Vaseline; higher alcohols such as cetyl alcohol, stearyl alcohol, and oleyl alcohol; protein hydrolysis products or derivatives thereof derived from animals and plants such as collagen, keratin, soybean protein, and wheat protein; solvents such as isopropanol, benzyl alcohol, ethanol and water; pH controllers such as sodium hydroxide, sodium hydrogen carbonate, ammonium hydrogen carbonate, ammonium monohydrogen phosphate, phosphoric acid, hydrochloric acid, succinic acid, citric acid, sodium citrate, and sodium pyrrolinate; antiseptic agents such as paraoxybenzoate esters and sodium dehydro acetate; polyhydric alcohols (humectants) such as propylene glycol and glycerine; chelating agents such as ethylenediamine tetraacetic acid; hair protecting ingredients, stabilizers, anti-inflammatory agents, coloring agents, antioxidants, emlulsifiers, perfumes, etc.

The hair dyeing agent of the invention may be in a formulation such as liquid, emulsion, cream, gel, paste, mousse, solid, powder or the like and can be formed also as an aerosol or spray.

Further, the method of coating used upon application to hairs also includes a method of coating by nozzle type, brush-like nozzle, or comb type.

Further, the hair dyeing agnet of the invention includes one-agent-type and two-agent-type hair dyes. In the case of the two-agent-type hair dye, the polyphenol derivatives of the invention is preferably contained in the second agent.

The invention utilizes a polyphenol derivative obtainable by sbjecting a polyphenol, or a polyphenol and an amino acid to a reaction using an alkaline solvent under the co-existents of oxygen molecules at pH during reaction of 6.5 or more and the present invention provides a hair cosmetic composition having an excellent oxidizing effect.

According to the invention, there can be provided a hair cosmetic composition having the same permanent wave oxidation fixing effect and a dyeing effect as in the existent case, causing less injuries to hairs and head hairs or disorders of skins and scalps without using an oxidizing agent such as hydrogen peroxide used so far. Then, it can protect or mend hairs during perming or hair dyeing operation. Further, the hair cosmetic composition of the invention can also provide an effect of removing malodors released from a reducing agent such as thioglycolic acid or cysteine used in the permanent wave agent, particularly, two-agent-type permanent wave first agent. Further, the polyphenol derivative of the invention is stable in the hair cosmetic composition.

Further, the polyphenol derivative of the invention also has an effect of directly acting on hairs per se and protecting hairs or mending hairs when it is incorporated into the hair cosmetic composition or hair care products such as shampoo, conditioner, hair rinse, hair dye, permanent wave agent, hair wax, hair cream, hair essence, hair lotion, hair spray, and hair mousse.

### Example

Although the present invention is to be described more specifically by way of examples, the invention is not at all limited thereto. In the followings, % means % by mass unless otherwise specified.

### Preparation Method of Polyphenol Derivatives

### Preparation Example 1

To the inside of a stirring vessel containing 1 L of an aqueous 0.05M solution of sodium carbonate (pH 11.2), 20 mmol of gallic acid was added and stirred vigorously at 25°C for 3 hours under the conditions that air communicates freely and the surface of the reaction solution can be in contact sufficiently with air. Then, the reaction solution was freeze-dried to obtain 9.06 g of a powder containing a polyphenol derivative (yield based on gallic acid of 241%).

### Preparation Example 2

To the inside of a stirring vessel containing 1 L of an aqueous 0.05M solution of sodium carbonate (pH 11.2), 20 mmol of gallic acid and 20 mmol of sodium glutamate were added and stirred vigorously at 25°C for 3 hours under the conditions that air communicates freely and the surface of the reaction solution can be in contact sufficiently with air. Then, the reaction solution was freeze-dried to obtain 12.8 g of a powder containing a polyphenol derivative (yield based on gallic acid of 340%).

### Preparation Example 3

To 80 g of dried tea leaves of green tea, 1,600 ml of an aqueous 50 mM solution of sodium carbonate was added and stirred vigorously at 30°C for 1 hour in a state capable of being in contact with air (pH of reaction solution: at 8.7). After filtration of the reaction solution, the filtrate was freeze-dried to obtain 51.8 g of a powder containing a polyphenol derivative (yield based on dry tea leaves of 64%).

### Preparation Example 4

To 100 g of raw coffee beans, 1,000 ml of an aqueous 50 mM solution of sodium carbonate (pH 11.2) was added, and stirred vigorously and extracted at 15°C for 3 hours in a state capable of being in contact with air (pH of reaction solution: at 7.8). After filtration of the reaction solution, the filtrate was freeze-dried to obtain 17.3 g of a powder containing a polyphenol derivative (yield based on raw coffee beans of 17.3%).

### Test Example 1 Permanent wave agent (two-agent-type)

### (Test method)

Hairs were protected at the ends with paper, wound around a plastic rod of 1 cm diameter, dipped in the following first agent, left at 40°C for 20 min, then rinsed sufficiently with water and subsequently dipped in each of the second agents described below at a room temperature for 15 min. After detaching the rod and water rinsing, they were dried on towel and then dried by a drier to confirm the wave effect. The results are shown in Table 2.

**Table 1 : First agent of permanent wave agent**

| Ingredient | Compounding amount |
|---|---|
| Ammonium thioglycolate (50% aqueous solution) | 10.00% |
| Aqueous ammonia (28%) | 3.00% |
| 1,3-butylene glycol | 2.00% |
| Methyl paraben | 0.20% |
| Purified water | 84.80% |

**Table 2: Second agent of permanent wave agent**

| Ingredient | 1-1 | 1-2 | 1-3 |
|---|---|---|---|
| Purified water | 100.00% | 94.00% | 90.00% |
| Sodium bromate | - | 6.00% | - |
| Polyphenol derivative of Preparation Example 3 | - | - | 10.00% |
| Wave effect | no | present | present |

When the polyphenol derivative of the invention was incorporated into the permanent wave second agent (1-3), wave effect for hairs was recognized.

### Test Example 2 Hair Dye

### (Test method)

One g of the hair color first agent and one g of the hair color second agent described below were mixed, applied to one g of a bundle of goat hairs of 10 cm length, left at a room temperature for 30 min, washed with water, shampooed, and then dried and the dyeing effect and finishing of hairs were confirmed. The evaluation criterion for the hair finishing is shown below. The results are shown in Table 4.

Evaluation criterion for hair finishing
○ :soft and easy to comb
Δ : somewhat poor in softness and combing
× : not soft and poor in combing as well

Judgment was conducted by five panelists.

**Table 3: Hair color first agent**

| Ingredient | Compounding amount |
|---|---|
| Paraphenylenediamine | 2.00% |
| Resorcine | 0.50% |
| Oleic acid | 20.00% |
| Polyoxyethylene oleyl ether (50 E.O.) | 3.00% |
| Diethanolamide stearate | 3.00% |
| Propyleneglycol | 12.00% |
| Isopropyl alcohol | 10.00% |
| Aqueous ammonia (28%) | 10.00% |
| Purified water | 39.20% |
| Sodium sulfite | 0.20% |
| Disodium edetate | 0.10% |

**Table 4: Hair color second agent**

| Ingredient | 2-1 | 2-2 | 2-3 |
|---|---|---|---|
| Purified water | 100.00% | 70.00% | 95.00% |
| Aqueous hydrogen peroxide (30%) | - | 30.00% | - |
| Polyphenol derivative of Preparation Example 4 | - | - | 5.00% |
| Hair dyeing effect | no | present | present |
| Finishing of hair | Δ | × | ○ |

### Example 1: Cysteine type permanent wave agent

Hairs were protected at the ends with paper, wound around a plastic rod of 1 cm diameter, dipped in a first agent, left at 40°C for 20 min, then rinsed sufficiently with water and subsequently dipped in a second agent at a room temperature for 15 min. After detaching the rod and water rinsing, it was dried on towel and then dried by a drier to confirm the wave effect. As a result, uniform wave was obtained from the base to the end of the hairs and the feeling of hairs was also satisfactory.

**Table 5 First agent**

| Ingredient | Compounding amount |
|---|---|
| L-cysteine hydrochloride salt monohydrate | 5.00% |
| Ammonia thioglycolate (50% aqueous solution) | 0.60% |
| Monoethanol amine | 3.00% |
| 1,3-butylene glycol | 2.00% |
| Methyl paraben | 0.20% |
| Purified water | 89.20% |

**Table 6: Second agent**

| Ingredient | Compounding amount |
|---|---|
| Purified water | balance |
| Polyphenol derivative of Preparation Example 1 | 10.00% |

### Example 2 Thioglycolic acid type permanent wave agent

A permanent wave treatment was conducted in the same manner as in Example 1 by using the first agent of Table 7 and the second agent of Table 8. As a result, uniform waves were obtained from the base to the ends of hairs and feeling of hairs was also favorable.

**Table 7: First agent**

| Ingredient | Compounding amount |
|---|---|
| Ammonium thioglycolate (50% aqueous solution) | 10.00% |
| Polyoxyethylene oleyl ether (10 E.O.) | 1.00% |
| Polyoxyethylene oleyl ether (20 E.O.) | 1.00% |
| Sodium lauryl sulfate | 0.50% |
| Disodium edetate | 1.00% |
| Aqueous ammonia (28%) | 5.00% |
| Purified water | balance |

**Table 8: Second agent**

| Ingredient | Compounding amount |
|---|---|
| Purified water | balance |
| Polyphenol derivative of Preparation Example 1 | 5.00% |

### Example 3 One-agent-type hair dyeing agent (cream type)

The following one-agent-type hair dyeing agent was applied on white hairs, treated at a room temperature for 30 min, washed with water, then shampooed and dried. The white hairs were died grayish color.

**Table 9: One agent-type hair dyeing agent (cream type)**

| Ingredient | Compounding amount |
|---|---|
| Paraphenylene diamine | 1.35% |
| Orthoaminophenol | 0.10% |
| Resorcine | 0.25% |
| Cetanol | 6.00% |
| Oleyl alcohol | 5.00% |
| Polyoxyethylene cetyl ether (15 E.O.) | 7.00% |
| Liquid paraffin | 10.00% |
| Chlorostearyl trimethyl ammonium | 1.00% |
| Propylene glycol | 2.00% |
| disodium edetate | 0.20% |
| Polyphenol derivative of Preparation Example 2 | 10.00% |
| Monoethanol amine, purified water | balance |

| | |
|---|---|
| (pH adjusted to 8.5 with ethanolamine) | |

### Example 4 One-agent-type hair dyeing agent (hair cream type)

The following one-agent-type hair dyeing agent was applied on white hairs, left for 10 to 30 min and then instantly washed or shampooed and, followed by drying. The treatment was conducted on every days for 10 days. As a result, white hairs were dyed to a grayish color.

**Table 10: One-agent-type hair dyeing agent (hair cream Type)**

| Ingredient | Compounding amount |
|---|---|
| Paraphenylene diamine | 0.14% |
| Orthoaminophenol | 0.01% |
| Resorcine | 0.02% |
| Cetanol | 6.00% |
| Oleyl alcohol | 5.00% |
| Polyoxyethylene cetyl ether (15 E.O.) | 7.00% |
| Polyoxyethylene cetyl ether (1.0 E.O.) | 3.50% |
| Liquid paraffin | 10.00% |
| Chlorostearyl trimethyl ammonium | 1.00% |
| Liquid lanoline | 1.00% |
| Disodium edetate | 0.20% |
| Ascorbic acid | 0.20% |
| Polyphenol derivative of Preparation Example 2 | 1.00% |
| Potassium hydroxide, purified water | balance |

| | |
|---|---|
| (pH adjusted to 7.5 with potassium hydroxide) | |

### Example 5: One-agent-type hair dyeing agent (treatment agent type)

The following mono-agent-type hair dyeing agent was applied on white hairs, treated at a room temperature for 30 min, washed water, then shampooed and dried. The white hairs were dyed to red-brownish color.

**Table 11: One agent-type hair dyeing agent (treatment agent type)**

| Ingredient | Compounding amount |
|---|---|
| Paraphenylene diamine | 0.04% |
| Orthoaminophenol | 0.02% |
| Nitroparaphenylene diamine | 0.20% |
| 2-amino-4-nitrophenol | 0.20% |
| Resorcine | 0.05% |
| Chlorocetyl trimethyl ammonium | 2.50% |
| Chlorostearyl trimethyl ammonium | 1.00% |
| Isopropyl myristate | 7.00% |
| Cetanol | 5.00% |
| Stearyl alcohol | 2.00% |
| Liquid paraffin | 4.00% |
| Liquid lanoline | 0.50% |
| Propylene glycol | 0.50% |
| Polyphenol derivative of Preparation Example 3 | 2.00% |
| Potassium hydride, purified water | balance |

| | |
|---|---|
| (pH adjusted to 7.5 with potassium hydroxide) | |

### Example 6: One-agent-type hair dyeing agent (treatment agent type)

In the same manner as in the existent hair treatment, the one-agent-type hair dyeing agent described in Table 11 was applied to a previously shampooed white hairs, treated at a room temperature for 5 to 10 min and then washed with water. When the treatment was conducted for 10 days, white hairs were dyed to red-brownish color.

### Example 7: Two-agent-type hair dye

A solution formed by mixing the following the hair color first agent and the second agent at 1:1 ratio was applied on white hairs, which were treated at a room temperature for 30 min, then washed with water, shampooed and dried. The white hairs were dyed to grayish color.

**Table 12: Hair color first agent**

| Ingredient | Compounding amount |
|---|---|
| Paraphenylenediamine | 2.00% |
| Resorcine | 0.50% |
| Oleic acid | 20.00% |
| Polyoxyethylene oleyl ether (10 E.O.) | 15.00% |
| Isopropyl alcohol | 10.00% |
| Aqueous ammonia (28%) | 10.00% |
| Purified water | balance |
| Sodium sulfite | 0.20% |
| Disodium edetate | 0.10% |

**Table 13: Hair color second agent**

| Ingredient | Compounding amount |
|---|---|
| Purified water | balance |
| Polyphenol derivative of Preparation Example 4 | 10.00% |
| Sodium sulfite | 0.20% |
| Disodium edetate | 0.10% |

### Industrial Applicability

The hair cosmetic composition according to the invention can treat hairs without causing damages to hairs and injuries of skins, and it has a permanent wave oxidation fixing effect and dyeing effect equal to or superior to that in usual.

The hair cosmetic composition of the invention can be used, for example, as a permanent wave oxidation fixing composition or a hair dye.

## Claims

1. A hair cosmetic composition, which comprises a polyphenol derivative obtainable by subjecting a polyphenol to a reaction using an alkaline solvent under the coexistence of an oxygen molecule at a pH during reaction of 6.5 or more.

2. The hair cosmetic composition according to claim 1, wherein the polyphenol is a polyphenol having an o-diphenol structure.

3. The hair cosmetic composition according to claim 1, wherein the polyphenol is a polyphenol having a p-diphenol structure.

4. The hair cosmetic composition according to any one of claims 1 to 3, wherein the polyphenol derivative is obtainable by further adding and reacting a divalent or multivalent metal ion.

5. The hair cosmetic composition according to any one of claims 1 to 4, wherein the polyphenol derivative is a polyphenol derivative obtainable by subjecting a plant extract which contains a polyphenol and does not substantially contains an amino acid to a reaction using an alkaline solvent under the coexistent of an oxygen molecule at a pH value during reaction of 6.5 or more.

6. The hair cosmetic composition according to any one of claims 1 to 5, wherein the polyphenol derivative is obtainable by further adding and reacting an amino acid.

7. A hair cosmetic composition, which comprises a polyphenol derivative obtainable by subjecting a plant extract and/or plant body containing a polyphenol and an amino acid to a reaction using an alkaline solvent under the coexistent of an oxygen molecule at a pH value during reaction of 6.5 or more.

8. The hair cosmetic composition according to any one of claims 1 to 7, wherein the hair cosmetic composition is an oxidative fixing composition for permanent wave.

9. A method of treating hairs with a polyphenol derivative obtainable by subjecting a polyphenol to a reaction using an alkaline solvent under the coexistence of an oxygen molecule at a pH during reaction of 6.5 or more.

10. A method of oxidatively fixing hairs during a permanent operation with a polyphenol derivative obtainable by subjecting a polyphenol to a reaction using an alkaline solvent under the coexistence of an oxygen molecule at a pH during reaction of 6.5 or more.
